(19) **Europäisches Patentamt European Patent Office Office européen des brevets**

(11) **EP 4 206 650 A1**

(12) **EUROPEAN PATENT APPLICATION**

(43) Date of publication:
**05.07.2023 Bulletin 2023/27**

(21) Application number: **21217899.0**

(22) Date of filing: **28.12.2021**

(51) International Patent Classification (IPC):
**G01N 21/21** $^{(2006.01)}$

(52) Cooperative Patent Classification (CPC):
**G01N 21/21**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **GERHARDT, Lutz Christian**
**Eindhoven (NL)**

• **HUIJBREGTS, Laurentia Johanna**
**Eindhoven (NL)**
• **DELLIMORE, Kiran Hamilton J.**
**Eindhoven (NL)**
• **VAN LIESHOUT, Ron Martinus Laurentius**
**Eindhoven (NL)**
• **PELSSERS, Eduard Gerard Marie**
**Eindhoven (NL)**
• **JOHNSON, Mark Thomas**
**Eindhoven (NL)**

(74) Representative: **Philips Intellectual Property & Standards**
**High Tech Campus 52**
**5656 AG Eindhoven (NL)**

(54) **SYSTEM FOR BODY FLUID ISOMER ANALYSIS**

(57)     A sensor device for measuring levels of one or more stereoisomers in body fluids, for example lactate in sweat. The device includes a body fluid collection means and an optical measurement system. The measurement of the isomer levels is based on measuring an optical property of an incident light which is known to be modified in a reliable and reproducible way by intrinsic optical activity of at least one isomer within the sample. By measuring the optical property, an indication of concentration of at least one stereoisomer can be derived. For example, D- and L-lactate are enantiomers and the optical activity of each has the effect of changing a rotation angle of light polarization. It also has the effect of changing a central frequency of a spectral composition of the incident light. In each case a polarization state modification is applied to the light before or after application to the sample.

FIG. 3

EP 4 206 650 A1

**Description**

FIELD OF THE INVENTION

This invention relates to a system for body fluid isomer analysis, for example for detecting lactate in sweat.

BACKGROUND OF THE INVENTION

Stereochemistry, stereoisomerism, or spatial isomerism, is a form of isomerism in which molecules have the same molecular formula and sequence of bonded atoms (constitution), but differ in the three-dimensional orientations of their atoms in space.

Enantiomers are a class of stereoisomers. Also known as optical isomers, enantiomers are two stereoisomers that are related to each other by a reflection: they are mirror images of each other that are non-superposable.

Many clinically relevant chemical compounds in body fluids are stereoisomeric, meaning that they exist in at least two stereoisomers. Examples include almost all naturally produced amino acids, e.g. alanine, lysine, proline, photo-isomers of bilirubin (e.g. produced during phototherapy), as well as metabolites such as lactate.

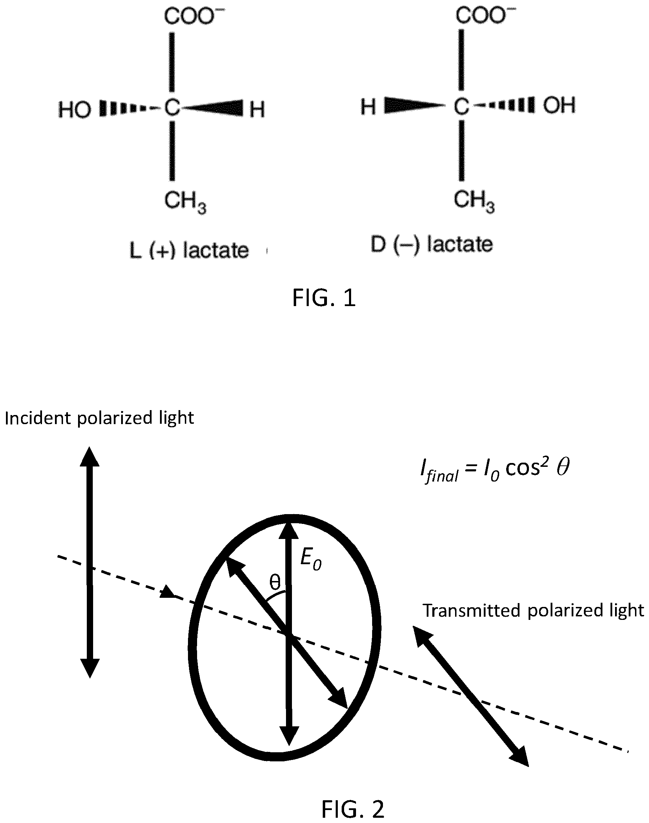

With regards to lactate, this is present in many body fluids including blood and sweat. It exists as two optical stereoisomers: L-lactate and D-lactate as shown in Fig. 1.

Two isomers are called enantiomers or optical isomers if they are mirror images of each other, have the same chemical and physical properties, and differ in their behavior toward the plane polarized light. Such molecules with mirror-image geometries are also called chiral. The mirror image of chiral compounds cannot be superimposed with the original. The mirror images are called left- and right-handed enantiomers. Chiral molecules are also characterized by their property to rotate the plane of polarized light.

Enantiomers are extremely important especially in biological and medicinal chemistry. For example, the pain reliever ibuprofen exists as two enantiomers, but only one of them is effective (the S-isomer) in treating pain. Also, the drug L-DOPA (L-dopamine) used for treatment of Parkinson's disease is effective only as the L- or R-enantiomer in the treatment.

With regards to lactate in particular, this is an emerging risk biomarker, for example for risk stratification for sepsis. More than 1.5 million people get sepsis each year in the US, of which at least 250,000 die. Measuring lactate levels has been used to risk stratify patients with suspected sepsis.

The predominant form of lactate in humans is L-lactate. Blood contains 50 to 100 times more L-lactate than D-lactate. Thus, in spot-check analyses, normally only L-lactate is measured while D-lactate (also carrying clinical information) is either not targeted or obscured by the dominant L-lactate concentration. The smaller amount of D-lactate that is normally present in the blood of humans (concentration 5-20 $\mu$mol/L in healthy adults compared to 1000 $\mu$mol/L for L-lactate) is solely derived from exogenous sources (diet and the carbohydrate-fermenting bacteria normally present in the gastrointestinal tract).

Blood serum lactate levels are traditionally low in normal individuals at rest. The reference range is around 1 $\pm$ 0.5 mmol/L and less than 2 mmol/L in critically ill patients; while studies performed on human lactate levels showed that an increase in lactate levels from 2.1 to 8 mmol/L at rest decreased the survival from 90% to 10%. Normal lactate level varies while elevated blood lactate concentrations are an early indication for sepsis.

If a patient has a serum lactate level >2 mmol/L at rest, either blood pressure and/or serum lactate level should be monitored. The most recent guidelines for severe sepsis or septic shock management indicate that a blood lactate concentration of greater than 4 mmol/L at rest mandates prompt resuscitation of severe sepsis or septic shock.

According to Guidelines for Management of Severe Sepsis and Septic Shock, blood lactate should be measured at least twice within 6 hours. A so-called racemic mixture of lactated Ringer's solution (14 mmol D-Lactate, 14 mmol L-lactate, 3.1 g/L) is usually given to patients after septic shock or with large fluid loss via an intravenous (IV) line at a rate of 30 mL/kg body weight/hour.

Also in healthy people, blood serum lactate levels can reach high values, namely during intensive exercise, due to too little oxygen in the muscles to cope with the energy demand. However, in this application, the focus is on people at rest.

There is usually a variable time lag between blood lactate and sweat lactate due to clearance effects and active/passive transport mechanisms. Therefore, ways to improve the clinical quality of sweat lactate measurements and to differentiate between D- and L-lactate is highly desirable.

Implementation of bedside lactate measurement in the emergency department has also been associated with reduced time to administration of intravenous fluids in patients with suspected sepsis and decreased rates of Intensive Care Unit admission and mortality. Measuring lactate levels supports identifying appropriate treatment of cardiac surgical patients, patients with acute kidney injury, and patients with community-acquired pneumonia.

Various methods and devices with different device properties and precision have been employed for D-and L-lactate detection such as the high-performance liquid chromatographic method (HPLC), colorimetric method, fluorescence method, spectrophotometric method and amperometric biosensors. Each method has advantages and limitations. Although the colorimetric method can realize a stable result, its sensitivity is relatively low. For the fluorescence method, the devices are relatively complex. For the spectrophotometric method, the process is relatively time-consuming.

Depending on the activity level, the sweat lactate concentration can be higher or lower than the corresponding blood lactate values.

There is therefore a need to improve the quality of sweat lactate measurement to achieve a clinically more meaningful measurement of sweat lactate in relation to blood. Lactate measurement may also be of interest for other conditions, such as providing an early indicator of ischemic decubitus.

As mentioned above, in spot-check analyses done in clinical labs, normally only L-lactate is measured using ELISA assays or blood-gas analyzers, while D-lactate (also carrying clinical information) is not targeted or obscured by the higher L-lactate content. Moreover, as in all circulating biomarkers, there is, usually a (variable) time lag between lactate in blood and sweat due to clearance effects and active/passive transport mechanisms (diffusion time from plasma to sweat gland, and transport kinetics of lactate over red cell membrane).

Therefore, a way to improve the quality of sweat lactate measurements and to differentiate between D-lactate and L-lactate (or to provide a measurement of the relative proportion of D/L-lactate concentration) is highly desirable as it can provide additional clinical value and diagnostic information. For example, such differentiation can enable the following use cases and can address unsolved clinical needs to enhance the accuracy and meaningfulness of sweat lactate measurements:

(i) Well-defined reference time point calibration for sweat lactate (sensor calibration) and the possibility to determine time lag between concentration changes in sweat and the corresponding changes in blood;
(ii) Establish a conversion factor/transfer function between the (lactate) concentration in blood and that in sweat; and
(iii) Direct measurement of D-lactate as indicator of the onset of infection, e.g., sepsis, infection, ischemia, traumatic shock or liver dysfunction.

SUMMARY OF THE INVENTION

The invention is defined by the independent claims. Dependent claims define advantageous embodiments.

According to examples in accordance with an aspect of the invention, there is provided a body fluid sensor comprising: a body fluid collection system; a polarized light source for directing analysis light to a collected body fluid sample; at least one polarization state modifier; a light detector; and a processor for processing the detected light.

During operation, the polarization state modifier and the collected body fluid sample are along a common optical path between the light source and the light detector. The processor is adapted to derive an optical property of the detected light which has been modified by a polarization modification induced by the body fluid sample, and thereby determine a concentration of at least a first stereoisomer of a pre-defined molecule in the body fluid sample, or a ratio between concentrations of at least a first and a second stereoisomer of the molecule present in the body fluid sample.

The polarization state modifier is arranged optically upstream from the sample, between the polarized light source and the sample.

The optical property determined by the processor is a polarization modification induced by an optical activity of the body fluid sample.

As will be described further below, embodiments of the invention provide an optical approach to measuring concentrations of stereoisomeric molecules in body fluids. This optical approach allows the method to be applied in a rapid, effectively real-time manner, and can be applied to different body fluids, and to the detection of any stereoisomeric molecule. Although the invention was originally motivated with a view to measuring lactate concentrations, and for example in sweat, as will become apparent, the solution created by the inventors can in fact be applied to measure concentrations of any stereoisomeric molecule, and any body fluid.

The body fluid may be sweat. However, other suitable body fluids may include for instance: urine, saliva, vaginal fluid and/or semen.

The at least first and second stereoisomers may be first and second enantiomers.

As mentioned, the at least first and second stereoisomers may be stereoisomers of lactate. In this case, the at least first stereoisomer may be D-lactate. The at least second stereoisomer may be L-lactate. Measurement of D-lactate can be used to provide an indicator of onset of infection, ischemia or traumatic shock.

The polarization modification induced by the optical activity of the sample may be a polarization rotation (that is, a change in the polarization angle). The polarization modification may be a change in the polarization linearity, e.g. a change from linear polarization to elliptical, or from circular to elliptical or vice versa. It may comprise a change in a degree of ellipticity or circularity. Whilst in specific embodiments described herein below, linearly polarized light is used, it should be realized that any of the other forms of polarization may be also advantageously applied.

D- and L-lactate are enantiomers (one class of stereoisomers), and each cause rotation of the plane of polarized light passing through them (optical activity) in opposite respective directions. The rotation is additive so that if the two enantiomers exist in the same solution, their optical activity sums to a net effective optical rotation. Thus, by measuring the net polarization rotation angle (angle of rotation of the polarization plane of linear polarized light induced by the solution), it is possible to determine the ratio of D- and L-lactate in the solution. This same approach will also work for any stereoisomeric molecule present in the probed fluid.

The net polarization rotation of the sample can be probed in different ways. One approach is to directly measure the change in polarization angle induced by the sample. This approach employs a polarimetry method. This is the method outlined above. A second approach is to use optical quantum weak measurement, wherein a change in the central frequency of the spectrum of the light is directly measured, where this is induced by the optical activity of the sample when the sample is illuminated with two perpendicularly polarized, and 180-degree phase shifted, beams. This approach will be outlined later. In both of these approaches, the same intrinsic physical property of the sample is being probed, i.e. the optical activity (polarization rotation) of the sample, but the two approaches each directly measure a different optical property of the detected light.

Methods described herein enable semi-continuous real-time measurement of two stereoisomers of a molecule, such as D- and L-lactate. The described sensor may in some cases include an integrated microfluidic sample collection system. Optical measurement embodiments described herein have the advantage that they do not require physical rotation of a polarizer element to detect isomers nor the use of reagents, nor capture surface regeneration. In some embodiments, the described sensor may be integrated in a body mounted device, such as an on-body or in-body sensor. For example, one embodiment proposes a wearable sweat lactate measurement device.

Various embodiments described herein enable more accurate and reliable stereoisomer measurements, potentially in wearable devices.

Some embodiments described herein enable translation or conversion from measured sweat stereoisomer concentrations to estimates of equivalent blood stereoisomer concentrations.

In some embodiments, the polarization state modifier is adapted to apply a set of different polarization rotation angles. This allows a net change in the polarization angle induced by the optical activity of the sample to be derived, by sensing the intensity of the light received at the detector after being subject to the different polarization rotation angles. The polarization state modifier may include a plurality of sections, wherein each section incorporates a polarization modifier which applies a different one of the plurality of different polarization rotation angles. This allows for the multiple polarization rotations to be applied without moving parts.

In some embodiments, the sensor may comprise a controller adapted to control the polarized light source for generating analysis light of at least two different wavelengths. The different wavelengths may be generated at different times, or an analysis light may be generated with a spectral composition which comprises a mix of the two or more wavelengths (i.e. the wavelengths are generated at the same time).

In some embodiments, the processor may be adapted to derive individual concentrations of the two stereoisomers of the molecule from measurement of the optical property (for example from measurement of the induced polarization rotation angle) for each of the at least two different wavelengths.

In some embodiments, the body fluid collection system may comprise a fluid conduit subsystem (comprising one or more fluid conduits), and wherein the sample is contained in the fluid conduit subsystem during analysis. The fluid conduit subsystem is configured such that the sample contained therein is optically accessible to the analysis light. The light passes through the conduit system containing the sample en route to the detector.

In some embodiments, the at least one polarization state modifier may be comprised by one or more walls of at least one fluid conduit of the fluid conduit subsystem.

The one or more walls of the fluid conduit subsystem are configured such that, in operation, there is an optical path into the sample via an at least portion of the wall which comprises the polarization state modifier.

The polarization state modifier may be a separate unit coupled to or integrated in the conduit wall, or the polarization state modifier may form the wall of the conduit. For example, in at least one set of embodiments, there may be provided at least one polarizer sheet which rolled into a tubular configuration to form the fluid conduit. By way of another example, there may be provided a fluid conduit formed of a material which has been pre-strained in manufacture to provide an intrinsic polarization modification behavior.

In some embodiments, the polarization state modifier may be imprinted on the wall of said at least one fluid conduit.

In some embodiments, the polarization state modifier may comprise a plurality of segments with different polarization angles along the fluid conduit subsystem and wherein the detector is configured to separately detect light that has passed through different segments.

In case that the polarization state modifier performs a different modification than a change in polarization angle, then the plurality of segments may each apply a different degree of the relevant modification, e.g. a different degree of change in polarization ellipticity or circularity. For example, the plurality of segments may each comprise a different respective wave plate with a different degree of rotation (e.g. formed of the same material, but with different thicknesses to provide more or less rotation).

The segments may be arranged for example such that, during operation, all simultaneously lie in at least one optical path extending from the light source to the detector via the sample in the fluid conduit subsystem.

In this way, the detector is able to obtain measurements for the multiple different polarization rotations without needing to physically move the polarization state modifier. Existing systems rely on actively rotating a single polarizer to obtain the measurements at different polarization angles. By providing an array of differently angled polarizers in the beam path, this active rotation can be avoided, enabling simplification and miniaturization of the sensor.

In some embodiments, a fluid flow channel defined by at least one conduit of the conduit system is arcuate, the arc of the conduit lying in a plane, and wherein said common optical path has a directional component which is perpendicular to said plane.

For example, the segments are arranged at consecutive locations along said arcuate fluid flow path.

This provides an efficient geometry for obtaining the multiple polarization angle measurements. An arcuate conduit is space efficient. It reduces the total width over which light must be directed compared to a linear conduit. It also allows in some embodiments an efficient construction, wherein the polarization state modifier is a continuous linear polarizer sheet, which is folded or rolled to conform to the curved geometry, whereupon the sheet inherently provides a series of different polarization angles relative to a light source having an optical path perpendicular to the plane of the arc.

The optical detector and light source can be on opposite sides of the sample or one the same side. For example, in one embodiment, the optical detector and light source are located on a same side of the fluid conduit subsystem, a reflector is provided at a location on an opposite side of the fluid conduit subsystem, and the reflector lies along the at least one optical path.

In some embodiments, the sensor may further comprise one or more electromagnetic coils for generating an electromagnetic field in an area, and wherein said common optical path passes through the electromagnetic field, for use in enhancing an angular resolution of the analysis light.

Above have been outlined certain advantageous features in accordance with a first main approach to probing the optical activity of the sample, using a polarimetry method. There will now be outlined below advantageous features in accordance with a second main approach to probing the optical activity, based on use of optical quantum weak measurement, wherein a change in a central frequency of the spectrum of light is measured.

As noted above, this approach shares in common with the first one that the underlying physical property upon which measurements are based is the same - namely the optical activity of the sample.

In accordance with one set of embodiments, there is provided a body fluid sensor comprising:

a body fluid collection system;
a polarized light source for directing analysis light to a collected body fluid sample;
a polarization state modifier;
a light detector; and
a processor for processing the detected light,
wherein, during operation, the polarization state modifier and the collected body fluid sample are along a common optical path between the light source and the light detector,
and wherein the processor is adapted to derive an optical property of the detected light which has been modified by a polarization modification induced by the body fluid sample, and thereby determine a concentration of at least a first stereoisomer of a predefined molecule in the body fluid sample, or a ratio between concentrations of at least a first and second stereoisomer of the molecule present in the body fluid sample,
wherein the polarization state modifier includes a birefringent element optically upstream from the sample and a linear polarizer optically downstream from the sample, and wherein the optical property determined by the processor is a change or shift in a central wavelength, $\delta\lambda$, of an optical spectrum the detected light compared to an optical spectrum of the light generated by the light source.

The at least first and second stereoisomers may be first and second enantiomers of the molecule.

In some embodiments, the at least first and second stereoisomers may be isomers of lactate. The at least first stereoisomer may be D-lactate. The at least second stereoisomer may be L-lactate.

The body fluid may be sweat. Other suitable body fluids may include for instance: urine, saliva, vaginal fluid and/or semen.

The birefringent element may be a half wave plate in some embodiments.

The polarization modification induced by the sample may be a polarization rotation in some examples. It may be caused by optical activity of the sample.

In some embodiments, the polarized light source may be linearly polarized in a first direction.

The birefringent element may be arranged to receive the light source and to output two linearly polarized light beams having respectively perpendicular polarization directions.

The linear polarizer may have a direction of polarization perpendicular to the first direction.

For example, the polarization state modifier may be understood as including first and second portions, one portion positioned optically upstream from the sample, the other positioned optically downstream from the sample, both arranged along a common optical path which passes through the sample, wherein the first portion comprises the birefringent element, and wherein the second portion comprises the linear polarizer.

The birefringent element may comprise a birefringent material (i.e. a material exhibiting birefringent properties).

The birefringent element may comprise an electrically addressable liquid crystal polarizer arrangement. In this case for example, the birefringent element is implemented through active electronic control of a liquid crystal unit, to induce a desired birefringent behavior from the unit.

In some embodiments, the body fluid collection system may comprise a fluid conduit subsystem, and wherein the body fluid sample is contained in the fluid conduit subsystem during analysis.

In some embodiments, the polarization state modifier may be comprised by one or more walls of at least one fluid conduit of the fluid conduit subsystem.

In some embodiments, the polarization state modifier may be imprinted on at least one wall of said at least one fluid conduit.

In some embodiments, a fluid flow channel defined by at least one fluid conduit of the fluid conduit subsystem is arcuate, the arc of the conduit lying in a plane, and wherein said common optical path between the light source and the detector has a directional component which is perpendicular to said plane.

In accordance with either the first main approach (polarimetry) or the second main approach (measurement of central wavelength shift) outlined above, the sensor may be provided integrated in a body-mountable unit. For example, this might be a wearable unit. For example, such a wearable unit may be configured such that, when worn, a fluid entry area of the body fluid collection system is held in fluid communication with a skin surface.

As mentioned above, certain embodiments in accordance with either the first main approach or the second main approach comprise a fluid conduit subsystem within which the body fluid sample is contained during analysis. The fluid conduit subsystem may include at least one fluid conduit for receiving a body fluid sample from an inlet, and a flow control means (e.g. a valve) for controlling a flow of the sample through the conduit. The conduit system may have an outlet area through which the received sample can be evacuated from the at least one conduit once analysis is complete.

Within the domain of body lactate measurement, useful clinical information relating to lactate absorption can be derived from measurement of a lag time between injection of a known quantity of lactate into the bloodstream, and detection of corresponding response features in the sweat lactate or other body fluid lactate measurement signal. There will now be outlined a proposed advantageous method for acquiring such information. This can be implemented using a sensor device in accordance with any of the embodiments or examples outlined above, or a different source of sweat lactate measurements can be used. The embodiments outlined above have the advantage that they enable rapid, real time, non invasive measurement, which is useful for acquiring repeated measurements at regular intervals. Alternatively however, the below method could instead be implemented using any other lactate measurement method such as use of another D- and L-lactate sensitive electrochemical sensor or biochemical capture surface (e.g. using Ab-Ag binding and ELISA binding kinetics to determine D- and L-lactate). Additionally, the same principle is also applicable to other stereoisomers found in the human body, such as amino acids.

Accordingly one or more embodiments provide a system comprising:

a body fluid sensor for measuring a concentration of at least one stereoisomer of a predefined molecule in a body fluid sample; and
a controller adapted to:

obtain from the sensor a series of body fluid stereoisomer concentration measurements for a subject, over a continuous measurement session;
detect, in the measurement series, timings of either positive or negative inflection points in the data series;
receive a data input indicative of timings of one or more stereoisomer injection events administered to the subject;
determine a lag time between at least one stereoisomer injection event and a temporally closest inflection point following the stereoisomer injection event.

The stereoisomer concentration measurements are measurements of a concentration of at least one stereoisomer of a predefined molecule in a body fluid of the subject. The stereoisomer injection events are administered injections to the patient of a quantity of the same molecule stereoisomer that is being measured (for example comprised by an injectable fluid).

The at least first and second stereoisomers may be first and second enantiomers of the molecule.

The at least first and second molecule stereoisomers may be isomers of lactate.

The at least first stereoisomer may be D-lactate. The at least second stereoisomer may be L-lactate.

The body fluid may be sweat. Other suitable body fluids may include for instance: urine, saliva, vaginal fluid and/or semen.

For example, at least one set of embodiment may provide a system comprising:

a lactate concentration sensor; and
a controller adapted to:

obtain from the sensor a series of lactate measurements for a subject, over a continuous measurement session;
detect, in the measurement series, timings of either positive or negative inflection points in the data series;
receive a data input indicative of timings of one or more lactate injection events administered to the subject;
determine a lag time between at least one lactate injection event and a temporally closest inflection point following the lactate injection event.

The sensor may in some examples be a sensor for detecting the lactate concentration in sweat.

The lag time between lactate administration and either a lactate peak or an upward inflection in the lactate provides useful clinical information relating to diffusion of the lactate to the sweat and excretion processes.

Timings of corresponding inflection points (positive or negative) in the blood lactate may also be measured, and the controller may determine a difference in the lag times between blood and sweat.

The above-described method can be provided as a separate aspect of the invention, in isolation from any particular system or apparatus for implementing it. In other words, a method may be provided comprising: obtaining a series of lactate or other molecule stereoisomer measurements for a subject, over a continuous measurement session; detecting in the measurement series timings of either positive or negative inflection points in the data series; receiving a data input indicative of timings of one or more lactate (or other molecule stereoisomer) injection events administered to the subject; and determining a lag time between at least one injection event and a temporally closest inflection point following the injection event.

Within the domain of body lactate measurement, it would be useful to be able to use sweat-derived lactate measurement (which is non-invasive and relatively fast) to provide an indicator of estimated corresponding blood lactate levels. Blood lactate levels are the more standard clinical parameter, and well understood by clinicians. Therefore, a conversion into an expected equivalent blood lactate concentration from a sweat lactate measurement would be very useful.

There will now be outlined a proposed advantageous method for performing such a conversion. This can be implemented using a sensor device in accordance with any of the embodiments or examples outlined above. Alternatively however, the below method could instead be implemented using any other molecule stereoisomer measurement method, such as for example (for lactate) use of another D-and L-lactate sensitive electrochemical sensor or biochemical capture surface (e.g. using Ab-Ag binding and ELISA binding kinetics to determine D- and L-lactate). Additionally, the same principle is also applicable to other stereoisomeric molecules found in the human body, such as amino acids.

According to one or more embodiments, there may be provided a system comprising:

a sweat sensor for measuring a concentration of at least one stereoisomer of a predefined molecule in a sweat sample (for example a sensor as described in embodiments earlier in this disclosure); and
a controller adapted to:

obtain from the sensor a first measurement of a concentration of the molecule stereoisomer of interest,
obtain from the sensor a second measurement of a concentration of the molecule stereoisomer of interest, where the second measurement follows administration of a stereoisomer injection to the subject of the stereoisomer of interest; and

determine a conversion factor, c, between a sweat concentration of the molecule stereoisomer and a blood concentration of the molecule stereoisomer by computing the following equation:

$$c = (S_{after} - S_{before})/(I \cdot V_i / V_b)$$

where c is the conversion factor from the sweat stereoisomer concentration to blood stereoisomer concentration, $S_{before}$ is the concentration of the stereoisomer of interest in sweat before the stereoisomer injection, $S_{after}$ is the stereoisomer concentration of the stereoisomer of interest in sweat after the stereisomer injection, I is the concentration of the stereoisomer of interest in the stereoisomer injection solution, $V_i$ is the volume of the stereoisomer solution injected, and $V_b$ is the blood volume of the subject.

For the specific example discussed above in which lactate is the molecule which is measured, then the stereoisomer of interest may be D-Lactate or L-lactate. The sweat concentration sensor is in this case a sensor for measuring lactate concentration in sweat (one or other or both of D-Lactate and L-lactate). The injection may be an injection of a D-Lactate or L-lactate solution, e.g. Ringer's solution.

Thus, more specifically, according to one or more embodiments, there may be provided a system comprising:

a sweat lactate sensor; and
a controller adapted to:

obtain from the sensor a first sweat lactate measurement of a lactate of interest, the lactate of interest comprising one or both of L- and D-lactate;
obtain from the sensor a second sweat lactate measurement of the lactate of interest, where the second measurement follows administration of a lactate injection to the subject of the lactate of interest; and
determine a conversion factor, c, between sweat L- or D-lactate and blood L- or D-lactate by computing the following equation:

$$c = (S_{after} - S_{before})/(I \cdot V_i / V_b)$$

where c is the conversion factor from sweat L- or D-lactate concentration to blood L- or D- lactate concentration, $S_{before}$ is the lactate concentration of the lactate of interest in sweat before the lactate injection, $S_{after}$ is the lactate concentration of the lactate of interest in sweat after the lactate injection, I is the concentration of the lactate of interest in the lactate injection solution, $V_i$ is the volume of the lactate solution injected, and $V_b$ is the blood volume of the subject.

By deriving a conversion factor, the sweat concentrations can be converted to blood concentrations, which avoids a clinician needing to wait for blood lab analysis.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows the chemical structure of D and L lactate;
Fig. 2 schematically illustrates Malus' Law;
Fig. 3 schematically illustrates an example body fluid measurement sensor in accordance with one or more embodiments;
Fig. 4 illustrates in more detail an example body fluid sensor in accordance with one or more embodiments;
Fig. 5 further illustrates the example body fluid sensor in accordance with one or more embodiments;
Fig. 6 illustrates light intensity as a function of applied polarization angle for samples of two different optical activities;
Fig. 7 shows a further example configuration for a sensor in accordance with one or more embodiments;
Fig. 8 schematically illustrates the effect of a half wave plate on the polarization state of light;
Fig. 9 schematically illustrates in exploded view the optical configuration of a body fluid sensor system in accordance with one or more embodiments, utilizing an optical weak measurement approach;
Fig. 10 illustrates detection of a central frequency shift in an optical spectrum of light, used to determine relative or absolute concentrations of stereoisomers of a molecule;
Fig. 11 illustrates schematically the blood lactate concentration after infusion of L- and D- lactate;
Figs. 12 and 13 illustrate the effect of multiple lactate infusions on D-/L lactate ratio in sweat: and
Fig. 14 illustrates determination of a time lag between blood and sweat lactate levels.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a sensor device for measuring levels of one or more molecule stereoisomers in body fluids, for example lactate stereoisomers in sweat. The device includes a body fluid collection means and an optical measurement system. The measurement of the isomer levels is based on measuring an optical property of an incident light which is known to be modified in a reliable and reproducible way by intrinsic optical activity of at least one isomer within the sample. By measuring the optical property, an indication of concentration of at least one stereoisomer of the molecule can be derived. For example, D- and L-lactate are enantiomers of lactate and the optical activity of each has the effect of changing a rotation angle of light polarization. It also has the effect of changing a central frequency of a spectral composition of the incident light. In each case a polarization state modification is applied to the light before or after application to the sample.

According to any embodiment described herein, the system may be adapted for measuring concentrations of at least one of a known pair of enantiomers which exist of a given molecule in a sample.

At least two main measurement approaches are possible in accordance with the invention, referred to as a first main approach and a second main approach, and each of these will be discussed in detail below.

The concept of the invention can be applied to measurement of any stereoisomeric molecule in any biological fluid.

In discussions below, embodiments will be described in particular which relate to measurement of D- and L-isomers of lactate within sweat. However, it should be understood that the same principles can be applied to measurement of D- and L-lactate in any other body fluid. Furthermore, as will be apparent, it should be understood that the same principles can be applied to measurement of any other molecule stereoisomer in either sweat or another bodily fluid.

In accordance with each of the first and second main measurement approaches mentioned above, embodiments propose a sensing device provided with an integrated optical sweat isomer analyzer system comprising: a fluid conduit assembly sub-system, at least one electromagnetic radiation (preferably light) polarization state modifier (e.g. a polarizer, wire grid polarizer, optical filter, retarder, wave plate, birefringent element); a polarized light source (e.g. one or more LEDs, superluminescent diode, laser, optical fiber-based illumination source), which may be mono-chrome or multi-color; and an optical detector such as for instance a light intensity-spectrum analyzer (e.g. photo-microspectrometer, spectrograph, polarimeter, Michelson interferometer).

The fluid conduit subsystem and polarization modifier is spatially arranged between the light detector and light source in such a way that all share at least one common optical axis in at least one dimension.

The device further includes a microcontroller which is adapted to convert the optical detector readings into stereoisomer measurement values. In some embodiments, the microcontroller may comprise a wireless communication module, e.g. Bluetooth, to enable output of the measurement values.

The device preferably further includes an integrated power or energy source (e.g. a battery or supercapacitor).

In some embodiments, an actuator may be included arranged for actuating a polarization state modifier to change the polarization state modification. The actuator could be a responsive-material based actuator in some embodiments.

The sensor system is configured and calibrated for measuring small concentrations of molecule stereoisomers in mixture and is able to differentiate optically polarizable mirror-molecules (such as D- and L-lactate).

The first main measurement approach will now be outlined in detail.

In this approach, the measurement principle of polarimetry is used to determine concentrations of stereoisomeric molecules in a body fluid. To illustrate the principles of this concept, an explanation will be provided below of a set of embodiments which detect D- and/or L-lactate concentrations in sweat. The same principle can be applied to other stereisomeric chemicals in other body fluids.

In the context of the present invention, D- and L-lactate can be characterized by their property to rotate the plane of polarized light. This property is called optical activity. The angle by which the polarization plane is rotated is called optical rotation, $\alpha$, and is measured in degrees of optical rotation (°OR). According to the direction in which the light is rotated, the enantiomer is referred to as dextrorotatory (d or +; Latin: right; clockwise) or levorotatory (1 or -; Latin: left; counterclockwise). The optical activity of enantiomers is additive. If different enantiomers coexist in one solution, their optical activity adds up to a net effective optical rotation.

From the optical rotation $\alpha$, the specific rotation $[\alpha]_\lambda^T$, which is a material constant of a given substance, can be derived. The specific rotation is the optical rotation for a given: concentration c, sample cell length /, temperature T, and wavelength $\lambda$, in accordance with Biot's law:

$$\alpha = \frac{[\alpha]_\lambda^T \cdot l \cdot c}{100}$$

By knowing the specific rotation, $[\alpha]_\lambda^T$, of a substance, the concentration ratio of D- and L- lactate can for instance be determined from a measurement of the optical rotation. The ratio of two enantiomers, as well as their purity and their concentration, can be analyzed by the application of polarimetry (at different wavelengths). For instance, the variance of specific rotation with wavelength, also known as optical rotatory dispersion, can be used to find the absolute concentration of molecules in a mixture.

As illustrated schematically in Fig. 2, the law of Malus states that the intensity of a beam of plane-polarized light after passing through a rotatable polarizer ($I_{final}$) varies as the square of the cosine of the angle ($\theta$) through which the polarizer is rotated from the position that gives maximum intensity ($I_0$):

$$I_{final} = I_0 \cos^2 \theta$$

In a standard polarimeter, the polarimetry principle is accomplished if a dissolved optically active substance is introduced between two polarizing filters, crossed at 90°. The intensity of the light on the detector optically downstream from the second polarizing filter varies as a function of the angular position of these two filters. At point zero, polarizer and analyzer are set in an angle of 90° towards each other, which means that no light reaches the detector (0% transmission). As soon as an optically active substance is introduced into the sample, the transmission will increase in dependence upon the plane of polarization. To measure the angle of the rotation induced by the sample, the analyzer is rotated up to a point where the transmission of the detector is again at a minimum.

Solutions or mixtures containing equal quantities (concentrations) of enantiomers are called racemic mixtures. Racemic mixtures are therefore optically inactive as the clockwise and counterclockwise optical rotations cancel each other out. Conversely, when one enantiomer is present in excess, a net rotation of the plane of polarization is observed.

At the limit, where all the molecules are of the same handedness, the substance may be said to be optically pure. Optical purity, or percent enantiomeric excess (ee), is defined as: Percent of major enantiomer - Percent of minor enantiomer. A material that is 50% optically pure contains 75% of one enantiomer and 25% of the other. If the specific rotation, $[\alpha]_\lambda^T$, of a pure chiral compound is known, it is possible to use the observed specific rotation to determine the optical purity of a sample of the compound:

$$Optical\ purity\ [\%] = \frac{[\alpha]_{Mixture}}{[\alpha]_{Pure\ enantiomer}} \times 100$$

An alternative definition of enantiomeric excess is given by:

$$Enantiomeric\ excess\ [\%] = \frac{A - B}{A + B} \times 100$$

where A and B are the masses of enantiomers A and B (e.g. D- and L-lactate).

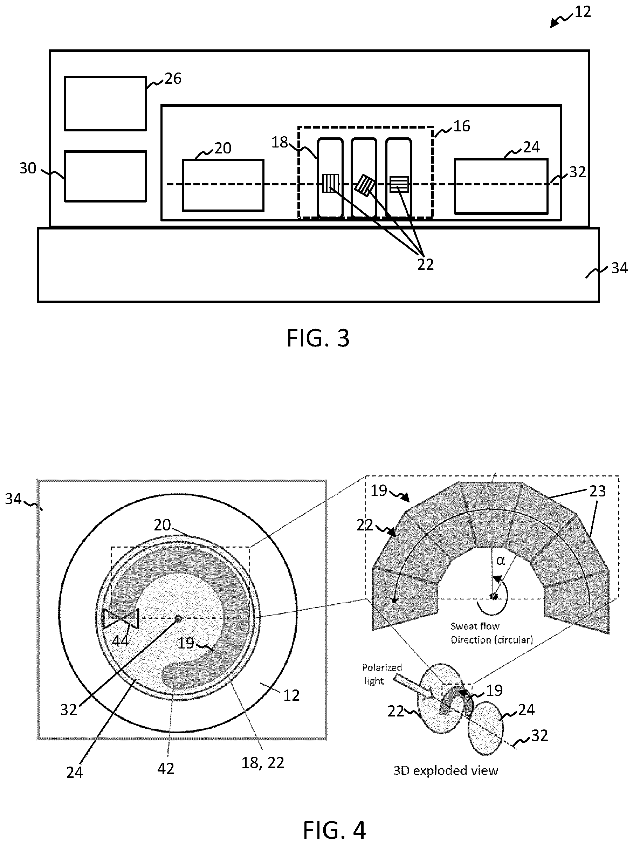

An example device in accordance with at least a first set of embodiments is shown in Fig. 3.

The device comprises a sweat sensor 12 comprising: a sweat collection system 16, a polarized light source 20 for directing analysis light to a collected sweat sample, and a light detector 24. The device further includes a polarization state modifier (arrangement) 22. At least during operation of the sensor, the polarization state modifier and the collected sweat sample are along a common optical path 32 between the light source 20 and the light detector 24. The polarization state modifier may be adapted to change an angle of the light polarization, in the case that the source light is linearly polarized. If the source light is non-linearly polarized, a different property of the polarization state may be changed, such as degree of polarization ellipticity (for elliptically polarized light), or degree of polarization circularity for circularly polarized light. More generally, the polarization state modifier should change a property or characteristic of the polarization state which is known to be modified by the optical activity of the sample.

In this example, the sweat collection system comprises a fluid conduit subsystem 18, wherein the sample is drawn into the fluid conduits of the conduit subsystem. Furthermore, in this particular example, the polarization state modifier 22 is arranged integrated in or on one or more walls of the conduits of the conduit subsystem, so that the light from the light source 20 passes through the polarization state modifier before passing through the sample in the conduit.

In some examples for instance, the polarization state modifier 22 may be formed by at least one polarizer sheet which is rolled up to define at least one of the fluid conduits. In some examples, at least one fluid conduit may be provided having a wall formed of a dichroic material layer, eg. polyvinylalcohol, which is pre-strained so as to provide an intrinsic polarization function due to directional mechanical stresses (material anisotropy) induced by the manufacturing.

These particular arrangements however are not essential. The sweat collection system can have a different arrangement for receiving and holding a sample, and the polarization state modifier could be a physically separate element. In general, the polarization state modifier 22 should be arranged optically upstream from the sample, between the polarized light source and the sample.

The sensor further comprises a processor 26 for processing the detected light.

During operation, the processor 26 is adapted to derive an optical property of the detected light which has been modified by optical activity of the sample (for example a polarization rotation induced by the sample), and thereby determine a concentration of D-Lactate in the sweat sample, or a ratio between D-Lactate and L-Lactate concentrations in the sample.

As mentioned, the optical property determined by the processor may be a polarization rotation angle induced by an optical activity of the sample.

The sensor device 12 may further include a local power or energy source 30 (e.g. battery, supercap) for powering the light source 20, light detector 24 and processor 26.

This illustrated example is shown in the form of an on-body sensor device, which is mountable directly on a skin surface 34, and wherein the sweat collection system is adapted to draw in a sweat sample directly from the skin surface. However, this is not essential.

Although this example device is described as being for use for a sweat sample, the same device could also be applied to any other body fluid sample, e.g. saliva, tear drops, urine, semen, vaginal fluid. The device can also be used to test body fluids of both humans and animals.

Furthermore, although this example device is described as being for determining concentrations of D-Lactate and L-Lactate, the device could also be used for detection of a concentration of a stereoisomer of any other molecule, or detection of a ratio between a concentration of a first stereoisomer and second stereoisomer of a given molecule. As will become clear from the explanations below, the principle of operation would work if applied to a substance containing any first and second stereoisomers of a given molecule, such as naturally produced amino acids, e.g. alanine, lysine, proline, and photo-isomers of bilirubin formed for example during phototherapy.

Furthermore, although this example device is described as utilizing an applied polarization modification in the form of a polarization rotation, this is not essential. The polarization state modifier 22 could apply a different kind of polarization modification such as birefringence. For example, a polarization modifier in the form of a birefringent material (crystals such as mica or quartz, liquid crystal polymer) can be used to produce vertically and horizontally polarized, phase-shifted light beams (i.e. convert input light into two well-defined polarized light beams.

An example device in accordance with at least one set of embodiments is shown in more detail in Fig. 4 Fig. 4 (left) shows a plan view (top-down view) of the sensor device. Fig. 4 (top right) shows a closer view of the fluid conduit of the fluid conduit subsystem 18 shown in Fig. 4 (left). Fig. 4 (bottom-right) shows a perspective view of the passage of the light through the series of optical components.

The sensor device 12 in this example has an arcuate geometry. The optical axis 32 previously identified in the schematic illustration of Fig. 3 extends out of the page from the perspective of Fig. 4 (left). A fluid flow channel defined by at least one conduit of the conduit system 18 is arcuate, the arc of the conduit lying in a plane, and wherein the common optical path 32 along which the light source, detector, polarization state modifier 22 and sample all lie has a directional component which is perpendicular to said plane.

The polarization state modifier 22 is comprised by one or more walls of at least one fluid conduit of the fluid conduit subsystem 18. For example, the polarization state modifier may be imprinted on the wall of said at least one fluid conduit.

The polarization state modifier 22 in this example is adapted to apply a set of different polarization rotation angles to light passing through it en route to the sample held in the conduit subsystem 18. The polarization state modifier 22 in particular comprises a plurality of segments 23 with different polarization angles along the fluid conduit subsystem, and wherein the detector is arranged and configured to separately detect light that has passed through different of the segments. The geometry of the device is such that the segments 23, during operation, all simultaneously lie in at least one optical path 32 extending from the light source 20 to the detector 24 via the sweat sample in the fluid conduit subsystem 18.

By way of further explanation, in accordance with at least one implementation, the polarization orientation (or optical rotation) of the light which passes through the sample at each of a series of locations along the fluid conduit, is configured by making use of segments 23 of polarizer filters or gratings (e.g. wire-grid polarizers) disposed on or in the walls of the microfluidic flow channel(s) of the conduit subsystem 18 (for example printed on the wall thereof) with varying polarization angles. The integration of these polarizer segments may be achieved during manufacturing using for example lithography or any other thin-film production or polarization filter production technique. Other examples for instance include a dichroic absorbing layer polarization filter, e.g. made from polyvinyl alcohol (PVA) plastic that is stretched during manufacturing. According to a further example, the polarization state modifier could be implemented by a liquid-crystal polarizer arrangement (e.g. a twisted nematic device, in-plane switching device, or fringe field switching device).

The fluid conduit subsystem 18 includes at least one fluid flow channel 19 the flow along which is controllable via a valve 44. The at least one fluid flow channel 19 includes an inlet 42 for inflow of sample fluid, e.g. sweat from the surface of the user's skin. The valve may be set so that sample fluid is continuously flowing through the flow channel, or the valve may be controllable to intermittently draw in fluid to make a measurement, and then evacuate the fluid from the flow channel after the measurement has been taken.

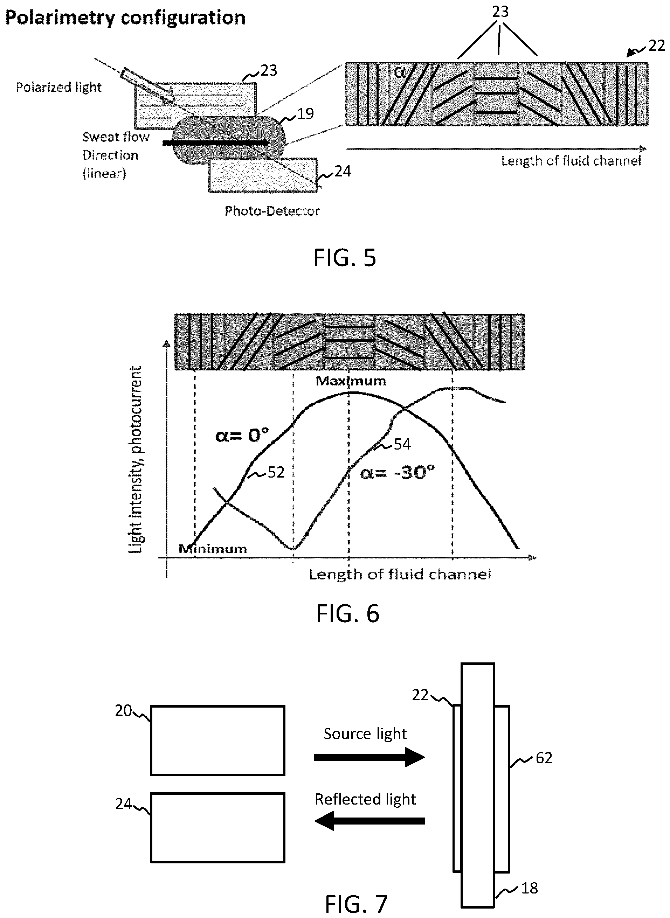

Fig. 5 (left) illustrates the passage of analysis light through one segment 23 of the polarization state modifier 22, and one corresponding section of the fluid flow channel 19. Fig. 5 (right) illustrates an example of the full length of the polarization state modifier 22, comprising a plurality of segments 23 which are arranged to be disposed at each of a series of consecutive locations along the length of the fluid channel 19.

Fig. 5 illustrates for example source light which is horizontally polarized by the illustrated segment 23 of the polarization state modifier before passing through the sample.

The set of different polarization rotation angles to be applied by the series of segments 23 may be determined based on performing iterative device optimization and calibration processes, taking into account clinically relevant D-Lactate and L-lactate mixtures (e.g. for lactate 0.1 - 10 g/mol) and the specific rotation of the mixtures or molecules of interest (or sweat samples which were analyzed through other methods such as ELISA and HPLC).

In operation, the light detector 24 is configured to detect an intensity of the light which has passed from the light source through the sample via each of the plurality of segments 23 of the polarization state modifier 22. Thus, a data series of light intensity measurements is obtained for different locations along the length of the fluid flow channel 19 (i.e. for polarization rotation angles applied by the polarization state modifier 22). The measurement can be done in a static state manner, i.e. without a need to physically move the polarization state modifier. For example, the detector may have an array of detection elements of sufficient size and arrangement to permit simultaneous sensing of the light portions which have passed through different of the polarization state modifier segments.

To determine from the acquired data series of light intensity measurements the concentration ratio of D-Lactate to L-lactate, the transmitted light intensity as function of location along the tube length (i.e. grating resolved light intensity) is plotted, as illustrated in Fig. 6. Either transmission minimum or transmission maximum (i.e. point of minimum intensity or maximum intensity) are identified or extracted from the plot. From this, the specific optical activity of the sample can be identified based on the known initial polarization of the source light. In other words, the net modification to the polarization (optical rotation, $\alpha$, in this case) induced by the (optical activity of) the sample can be determined based on knowledge of the initial polarization of the source light and the maximum and/or minimum intensity on the plot of intensity vs polarization rotation angle. From this, the ratio of the two enantiomers D-Lactate and L-lactate can be determined based on prior knowledge of their respective optical activities.

For example, the concentration determination operation performed by the processor 26 can be calibrated in advance according to known D-Lactate and L-lactate solution mixtures and known light wavelengths to determine suitable polarizer 22 angle ranges and suitable reference spectra to be able to convert the obtained intensity measurement signal to relative and absolute lactate concentration or D-to-L concentration ratios.

With reference to Fig. 6, this shows example plots of light intensity as a function of location along the length of the fluid channel (i.e. as a function of different polarization rotations applied by the polarization state modifier 22), for two samples exhibiting two different respective optical rotations. The first plot 52 is for a sample exhibiting an optical rotation of $\alpha = 0°$, and the second plot 54 is for a sample exhibiting an optical rotation of $\alpha = -30°$. Either the transmission maximum or minimum + 90 degree can be used to determine the net optical rotation of the sample, and from this a ratio of isomer concentrations can be determined, based on calibration data stored in a memory of the processor 26. The calibration data for example comprises a pre-determined transfer function for mapping measured sample net optical rotation to a ratio of D-Lactate to L-lactate.

Different wavelengths of source light are associated with different observable functions of optical rotation vs D-L lactate concentration ratio, when applied to a given sample. If these different per-wavelength functions are known, then absolute concentration of D-Lactate and/or L-Lactate can be determined by identifying intersections of the measured intensity plots for each of a plurality of applied wavelengths of light.

According to at least one variant of the above-described embodiment, the polarized source light additionally passes through an electromagnetic coil arrangement comprising one or more electromagnetic coils for generating an electromagnetic field. This arrangement is known as a Faraday modulator. This can further enhance the angular resolution before it is coupled into the sweat sample. Thereby an oscillation is superimposed onto the plane of polarization by means of the Faraday effect. The Faraday modulator may be positioned between the sweat sample and light source (before the light passes through the fluid channel 19).

According to at least one variant, and as illustrated in Fig. 7 the (polarized) light source 20 and light detector 24 may be arranged on a same side of the conduit subsystem 18, with a reflector 62 at a location on an opposite side of the fluid conduit subsystem, and wherein the reflector lies along the at least one optical path from the light source to the light detector via the sample. The reflector is a specular reflector for example. The reflector 62 may be a separate component, or may be integrated on or in the wall of the fluid channel (e.g. as a metallic coating acting as a reflective mirror). This arrangement has the advantage of being more compact. As shown in the illustrated example of Fig. 7, the polarization state modifier 22 is preferably arranged on a single side of the fluid conduit subsystem 18, preferably between the polarized light source 20 and the sample.

A second main approach to measuring molecule stereoisomer concentrations in a body fluid sample will now be outlined in detail.

The second main approach is based on the principle of optical quantum weak measurement.

In accordance with this second main measurement approach, an additional polarization modifier, in the form of a birefringent element (e.g. a birefringent material such as mica or quartz crystals, or a liquid crystal polymer) is used to produce vertically and horizontally polarized, phase-shifted light beams (i.e. convert input light into two well-defined polarized light beams).

Birefringence is the optical property of a material having a refractive index that depends upon the polarization and propagation direction of light. This optical anisotropy is responsible for the phenomenon of double refraction, whereby a ray of light, when incident upon a birefringent material, is split by polarization into two rays taking slightly different optical paths. When light passes through a birefringent material, one polarization is subject to a greater delay than the other. This changes the relative phase of the fields, and consequently changes the polarization.

In a birefringent material, one polarization travels faster than the other and phase-shifts slow axis polarizations by a half or quarter wavelength (i.e. 90° or 180° phase shift), relative to the other polarization. This eventually decomposes an initially linearly polarized light wave into two (phase-shifted) components, or converts an initially linearly polarized light wave into circular or elliptically polarized components.

The phenomenon of birefringence is used in so-called retarders (or waveplates) that alter the polarization state of light in a manner that depends upon the retardance of the retarder and upon the angle between the retarder fast axis and the input plane of polarization. One polarization component travels through the material with different velocity (due to birefringence) and is phase shifted relative to the other, producing a modified polarization state.

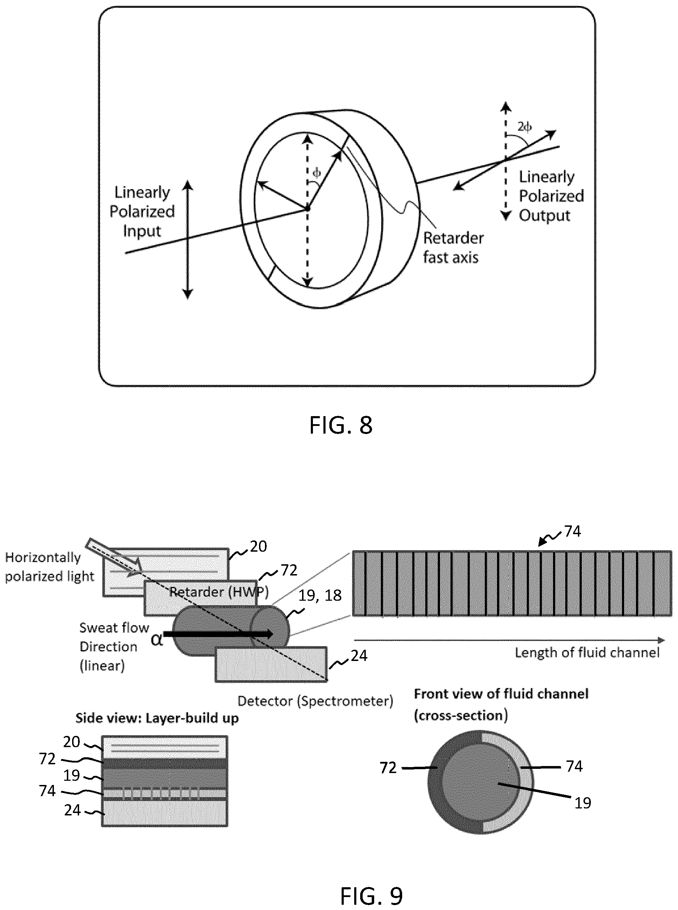

For example, half-wave retarders (or half wave plates) are sometimes called polarization rotators. An example is schematically illustrated in Fig. 8.

A half-wave retarder flips the polarization direction of incoming light relative to the retarder fast axis. When the angle between the retarder fast axis and the input plane of polarization is 45°, horizontal polarized light is converted to vertical polarized light. A half-wave retarder rotates a linearly polarized input light by twice the angle between the retarder fast axis and the input plane of polarization, as shown in Fig. 8. A retarder (or waveplate) resolves a light wave into two orthogonal linearly polarized components and produces a phase shift between them. The resulting light wave is generally of a different polarization form. Ideally, retarders do not polarize, nor do they induce an intensity change in the light beam, they simply change its polarization form.

In general, wave-plates are polarization converters through which one polarization direction propagates faster. A half-wave plate (HWP) can rotate the plane of polarization from 0-90 degree without any loss of intensity (creating a combination of vertically and horizontally polarized light with a phase-delay of 180 degrees). The fast axis of the HWP is at 45° relative to the input polarization direction.

The principle of birefringence is utilized in accordance with at least one set of embodiments which operates according to a second main measurement approach. The general structure and operation of the body fluid sensor according to this set of embodiments may be as follows. Again, although this example is described in terms of measurement performed on a sweat sample, the same device may be applied to any other bodily fluid. Also, although the device is described as being used for measuring D-Lactate and/or L-lactate concentrations, the same optical principles may be used for measuring concentration of first and/or second stereoisomers of any other molecule.

A sweat sensor is provided which comprises: a sweat collection system; a polarized light source for directing analysis light to a collected sweat sample; a polarization state modifier; a light detector; and a processor for processing detected light.

During operation, the polarization state modifier and the collected sweat sample are along a common optical path between the light source and the light detector.

The processor is adapted to derive an optical property of the detected light which has been modified by a polarization modification induced by the sweat sample, and thereby determine a concentration of D-lactate in the sweat sample, or a ratio between D-lactate and L-lactate in the sweat sample.

For instance, with reference to Fig. 9, the polarization state modifier 22 includes a birefringent element 72 optically upstream from the sample and a linear polarizer 74 optically downstream from the sample, and wherein the optical property determined by the processor is a change in a central wavelength, $\delta\lambda$, of an optical spectrum the detected light compared to an optical spectrum of the analysis light generated by the light source.

In particular, in preferred examples, the polarized light source is linearly polarized in a first direction (e.g. using a first polarizer element), the half wave plate is arranged to receive the light source and to output two linearly polarized light beams having respectively perpendicular polarization directions; and wherein the linear polarizer has a direction of polarization perpendicular to the aforementioned first direction.

The general structure and arrangement of the sensor device may be substantially similar to that shown in Fig. 3, except for a change in the polarization state modifier and in the processing operation performed by the processor to derive the stereoisomer measurements. For example, the sample collection system comprising the fluid conduit subsystem 18 and the light source may the same. The light detector is a multi-frequency light detector, whereas this is not essential for the first measurement approach previously described.

The arrangement of optical components according to the present set of embodiments is shown schematically in exploded form in Fig. 9. Fig 9 (top left) shows an exploded view of the optical components along the optical path from the light source 20 to the light detector 24 via a birefringent element 72 (e.g. a retarder or half wave plate) arranged optically upstream from a sample contained in a fluid flow channel 19 of a fluid conduit subsystem 18. The source light 20 is linearly polarized (horizontally polarized for the purposes of this example). This initial linear polarization could be achieved using a separate linear polarizer arranged between the light source and the birefringent element 72, or the light source could have integrated polarization means. A linear polarizer with an orthogonal direction or polarization to the source light (i.e. vertical polarization in this case) is arranged optically downstream from the sample in the fluid channel 19, between the sample and the light detector 24. In this example, the linear polarizer 74 is integrated in or on a wall of the fluid channel which holds the sample, as illustrated schematically in Fig. 9 (top-right).

The light detector may be a spectrometer in this example.

In more detail, according to at least one set of embodiments, the birefringent element 72 provides a polarization rotor and is placed between the sample in the conduit subsystem 18 and the light source 20 and used to produce a pair of vertically and horizontally polarized, phase-shifted light beams (i.e. converts the input polarized light into two polarized light beams).

Optionally, one or more of the optical components may be integrated as part of the wall(s) of one or more fluid channels 19 of the fluid conduit subsystem 18. In some examples, just the linear polarizer 74 may be integrated as part of the wall of a fluid channel. In other examples, both the birefringent element 72 and the linear polarizer may be integrated in walls of the fluid conduit subsystem. The latter option is illustrated schematically in Fig. 9 (bottom right) which shows how the birefringent element 72 and the linear polarizer could be integrated in opposite wall portions of each fluid channel 19 of the fluid conduit subsystem. Such a configuration might be manufactured for example using lithography or any other thin-film technology (e.g. electron beam, inverse microfabrication, lift-off micro-structuring), using for example mica as a substrate.

The birefringent element may comprise a birefringent material. Alternatively, the birefringent element may comprise an electrically addressable liquid crystal polarizer arrangement (polarization rotator). The birefringent properties of the liquid crystal polarizer may be controllable, for example by the processor, or a different controller component. By way of one example, the fluid conduit subsystem 18 may be arranged in-between two transparent electrodes, and wherein the liquid crystal is confined between one of the electrodes and the one or more conduits 19.

To explain the optical principle of operation further, in accordance with this set of embodiments linearly polarized input light is decomposed into horizontally and vertically polarized light components, and phase-shifted/modulated by 180 degrees using a birefringent element such as a half-wave plate (HWP). This optical measurement procedure is analogous to a so-called weak measurement configuration, as outlined for example in the following paper: Qiao et al., The real-time determination of D- and L-lactate based on optical weak measurement. Anal. Methods, 2019, 11, 2223-2230.

According to the theory of weak measurement, where there exists a phase modulation between two eigenstates related to the system, a shift of the central wavelength of the output optical spectrum bears a close relationship to the angle of pre-selected polarization in the frequency domain. Hence, by altering the pre-selected polarization state using an applied optical rotation, a concentration ratio of two enantiomers can be detected, as the optical weak measurement makes use of spectral or frequency analysis of quantum-mechanical states of polarized light.

In more simple terms, in a weak optical/light quantum mechanical measurement, an analysis of the light momentum of two polarized light beams can be achieved in a frequency domain by comparing a preselected quantum mechanical polarization state with a post-selected state. The preselected state is established through a first polarizer (integrated with the light source 20 or provided immediately optically downstream from the light source) in combination with the birefringent element (e.g. half wave plate), while the post-selected state is generated by a second polarizer (the linear polarizer 74 previously discussed). A measurement is referred to as 'weak' when the spread in the wavelength bandwidth (to analyze the states) is larger than the shift or phase difference of the polarization states (caused by optical rotation), i.e., the two polarized light beams cannot be completely separated in space. The phase difference between the two polarizations caused by optical rotation of isomers is measured by weak value amplification.

A weak quantum measurement provides the advantage of higher sensitivity at the price of lower detection probability. Since the post-selection state is almost orthogonal to the pre-selection state (e.g. two crossed polarizers), the photons received by the detector 24 are significantly reduced. To maintain a good signal-to-noise ratio, a higher power light source, such as a super-luminescent diode, or a longer integration time may be used.

As mentioned above, in a weak measurement system, there is a weak coupling between the measuring device and the system. The perturbation to the system induced by the change of the physical parameter under test (e.g. change of optical rotation due to optical activity of the isomers being probed) produces a (phase) difference between the eigenstates in the momentum space (frequency domain) or position space (time domain).

Using the purely imaginary weak value, combined with a frequency-domain analysis, it is possible to determine the optical activity of the isomers under investigation by quantitatively analyzing the shift of the central wavelength $\delta\lambda$ of the output spectra. This shift (towards lower or higher center wavelengths depending on lactate-isomer mixture or a concentration relative to a racemic reference mixture) is used as a proxy for the optical rotation $\alpha$ of the sample, and can be measured in practice using an optical detector comprising a micro-spectrophotometer for example.

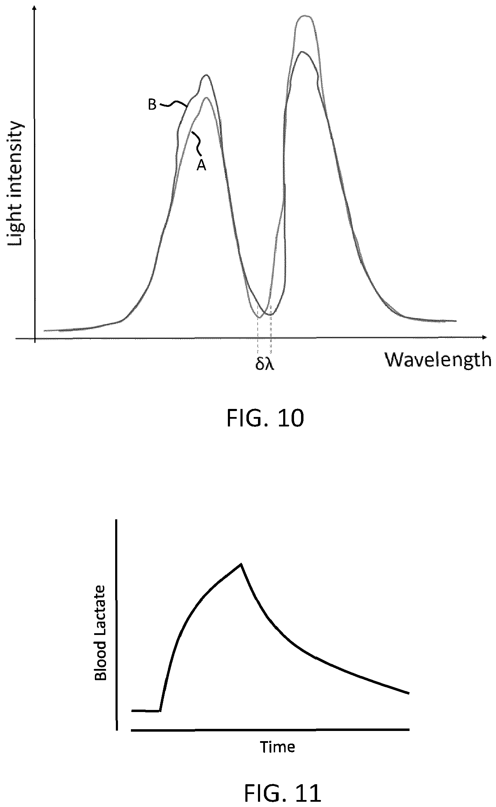

An example of the measurement principle is shown in Fig. 10 which illustrates a schematic example of two optical weak measurement spectra for two different samples, each having a different concentration of a same given isomer of interest. The change of the center wavelength towards lower or higher center wavelengths, relative to a (racemic) reference mixture, is the characteristic feature to differentiate between D-and L-lactate or any other isomer compounds.

As illustrated by Qiao et al. (cited above), the two lactate enantiomers (D- and L-lactate) induce respectively opposite shifts in the wavelength (indicative of their opposite optical activities). In particular, increasing the concentration of D-lactate causes a negative wavelength shift, while increasing concentration of L-lactate causes a positive wavelength shift.

By measuring the net shift in the central wavelength of the measured optical spectrum, the net optical activity, and/or the net concentration ratio between D and L lactate, can be derived using for example a pre-determined conversion or translation function or lookup table.

The two main measurement approaches outlined above can be directly used to identify a ratio between the concentration of D-Lactate and L-lactate. A relationship between this ratio and either the measured polarization rotation at which an intensity peak occurs (first measurement approach) or the shift in the central wavelength of the output light spectrum (second measurement approach) can be determined in advance and pre-stored in a memory of the processor 26.

For determining absolute D-lactate and/or L-lactate concentrations, several options are possible, as outlined below.

One option is to measure a molar absorption coefficient of the sample or measure a total light absorption by the sample as a function of wavelength, using Lambert-Beer's law, and use this to derive a decomposition of the sample into individual contributions to the absorption by one or more components of the sample mixture. The properties of the standard Lambert-Beer law are valid even if more than one material absorbs light in the medium. Each absorber contributes to the total absorbance and the resulting total absorbance A is a superposition of the individual absorbance processes. Based on this, it is possible to determine absolute concentrations of the individual components of a biofluid mixture comprising multiple components, e.g. using multi-variable regression analysis.

Another option is to include in the sensor device 12 an additional conventional L-lactate sensor (e.g. an electrochemical sensor), which allows conversion of the L/D lactate concentration ratio into absolute L-lactate and absolute D-lactate concentrations. In other words, the additional sensor provides the absolute L-lactate concentration (which is easier to detect due to its higher levels in biofluids), and using the ratio, the D-lactate concentration can accordingly be derived.

Another option is to include additional sensing means for measuring a refractive index of the sample. The refractive index may be used to provide an indication of the absolute total concentration of D- and L-lactate combined (using a pre-determined transfer function). Using the D/L lactate concentration ratio, the individual absolute D- and L-lactate concentrations can be derived.

There will now be described a first and second further method employing use of body lactate measurements for obtaining further clinically relevant information.

It should be noted out that these first and second methods described below are not necessarily limited to the use of any of the example sensor devices described previously in this disclosure. In general, the D- and/or L-lactate measurements could be derived from any suitable means, for example D- and L-lactate sensitive electrochemical sensors, or capture surfaces. Capture surfaces provide an alternative sensing means using Ab-Ag binding and ELISA binding kinetics. However, the two main optical approaches described previously are preferred since they are simpler, faster, and allow for greater measurement frequency (e.g. semi-continuous). For example, alternative approaches require reagent replenishment and capture surface regeneration.

In accordance with one or more embodiments of at least one aspect of the invention, there is provided a method comprising the following steps:

obtaining a data series of lactate measurements for a subject, over a continuous measurement session;

detecting, in the measurement series, timings of either positive or negative inflection points in the data series;

receiving a data input indicative of timings of one or more lactate injection or infusion events administered to the subject; and

determining a lag time between at least one lactate injection or infusion event and a temporally closest inflection point following the lactate injection or infusion event.

The lag time is indicative of rate of diffusion of the injected lactate into the body which can be clinically relevant, for example for diagnosing sepsis or predicting sepsis onset events.

There may be provided a processor adapted to perform the method. There may be provided a computer program product comprising code means configured, when run on a processor, to cause the processor to perform the method.

There may be provided a system comprising: a sweat or other biofluid lactate sensor, for example in accordance with any of the examples or embodiments outlined previously in this disclosure; and a controller or processor adapted to perform the method using lactate measurements obtained from the aforementioned sensor.

In this embodiment, lactate concentration measurements are used as a reference point calibration, for instance in connection with sepsis detection after surgery. In this use case, preferably either one or more racemic Ringer's solution infusions (14 mmol D-lactate, 14 mmol L-lactate) or one or more D-lactate injections are given and the onset of one or more characteristic transient peaks in sweat lactate concentration are observed. Optionally these may be used for correlating the sweat lactate measurement with corresponding blood lactate values.

Since D-lactate is produced by the body itself in only negligible quantities (roughly 100 times less than L-lactate), for purposes of monitoring uptake and diffusion of an injected lactate sample, it is advantageous to use D-Lactate, since body-produced D-lactate will not interfere with measurements. It is proposed in some embodiments to measure the D-lactate concentration in sweat or alternatively the D-/L or L-/D lactate ratio. Due to uptake dynamics and transport processes in the body, there may be some time lag, $\Delta t$, between the administration of the Ringer's solution, and the measurement of the D-lactate response in sweat. The baseline concentration of D-lactate in the body is assumed to be zero, due to the aforementioned negligible production in the body. After injection or infusion of the Ringer's solution the D-lactate concentration will rise until it reaches a peak concentration. Thereafter it will decay exponentially to zero, due to clearance from the body, unless further Ringer's solution or D-lactate is administered. This characteristic peaking pattern is illustrated schematically in Fig. 11.

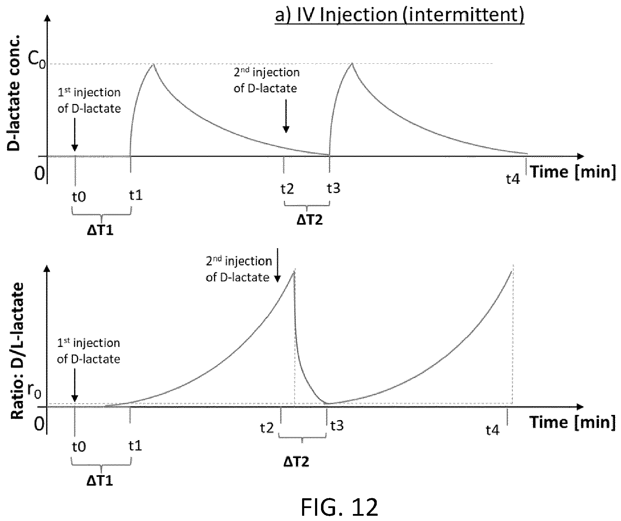

As illustrated in Fig. 12, when multiple, consecutive infusions of Ringer's solution or injections of D-lactate are administered, the inventors have recognized that determining the precise timing of the inflection or maximum point may be challenging due to the intermittent (i.e. semi-continuous) nature of sweat measurement. Nevertheless, the time difference, $\Delta T$, between the start of infusion or injection and a change in the slope from a decreasing to an increasing trend in D-lactate concentration, D-/L or L-/D lactate ratio can be measured. In other words, an inflection point (change in direction of the slope) is detected. Thus, in summary, either a negative or positive inflection point can be measured, corresponding to either a peak or sudden rise or sudden drop in lactate respectively.

With reference to Fig. 12, this shows measurement of the time lag between each of two discrete lactate injections and corresponding sweat lactate response, with the lactate levels allowed to return to baseline before second injection is administered. The time lag $\Delta T1$ between the first D-Lactate injection and the sweat lactate response (in the form of a positive inflection in the lactate signal), and the lag time $\Delta T2$ between the second D-lactate injection and the corresponding sweat lactate response (in the form of a positive inflection point) are both shown in Fig. 12.

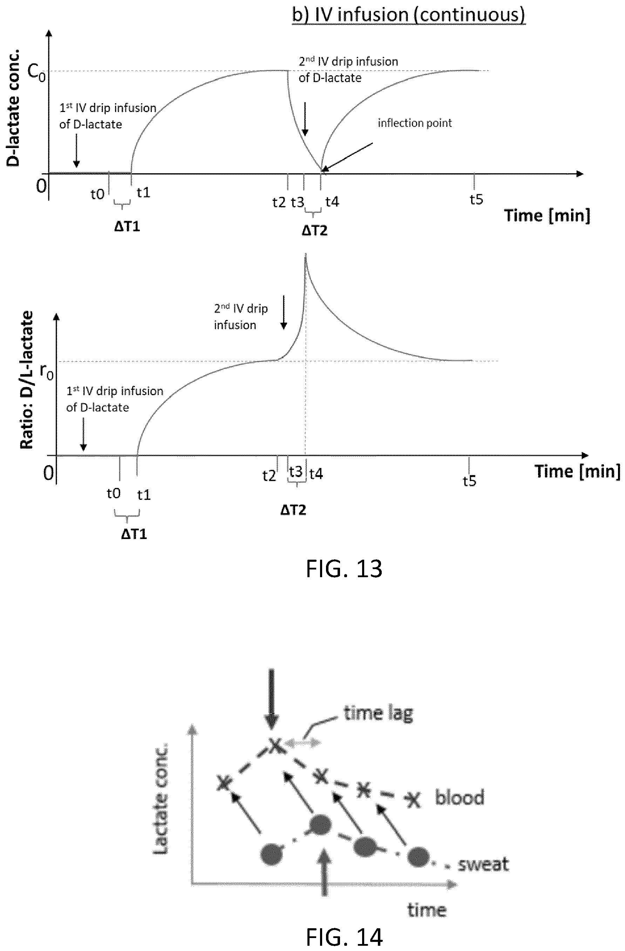

Fig. 13 shows a further example in which a plurality of lactate infusions are administered, each lasting for a certain time duration. The time difference $\Delta T1$ and $\Delta T2$ between the end of each lactate infusion and a corresponding lactate response in the sweat lactate signal (in the form of an inflection in the signal) is shown. Due to bodily uptake of the lactate, the D/L lactate ratio should initially continue to drop after the second infusion, but then start increasing again, leading to an inflection point. Here the lactate solution is assumed to be administered before complete clearance has occurred, i.e. the return to the baseline ratio does not occur.

As noted above, L-lactate is produced naturally by the body at much higher concentration levels than D-lactate. Nonetheless, the rate of endogenous production of L-lactate is quite low (for example 0.85 mol/(70 kg Body Weight $\times$ 24 hours)), which means that it will have a negligible influence on the measured D-/L or L-/D lactate ratio. It is also to be noted that the reported clearance for infused L-lactate is 13.1 ml/(Body Weight * Time) compared to 9.3 ml/(Body Weight $\times$ Time) for infused D-lactate. The slightly slower clearance time of infused D-lactate means that the ratio of D-/L-lactate will asymptote to $\infty$ as the D- and L-lactate clear, while in contrast the ratio of L-/D-lactate will asymptote to 0 as the D- and L-lactate clear. It may in fact be more advantageous to use the D-/L-lactate ratio rather than L-/D-lactate, since the latter ratio will result in very low values which may be more difficult to measure.

It is noted that while the Ringer's solution is widely used for intravenous infusion it is also possible to infuse or inject higher or lower concentrations of D-lactate, or different ratios of D-/L-lactate (1:2, 1:4, 2: 1, 4:1, etc.). In these cases, the metabolization (including clearance and/or production) of D- and L-lactate may change significantly. However, the basic underlying principle of this embodiment will remain unchanged.

Determining the lag time between lactate injection/infusion, and sweat lactate response is clinically useful in a number of ways.

It is firstly useful as a general biomarker. Changes in lactate levels are the sum of ongoing production and removal from the blood by excretion (clearance via e.g. urine or sweat) and its metabolism (e.g. uptake by cells as a direct source of energy, conversion to glucose by the liver). The lag time or different length of lag times may also give potentially indirect information on the renal filtration rate (renal clearance capacity) and allow for monitoring kidney function.

Further to this, the above described principle of dynamic reference point calibration may also provide a means for determining a time lag response between sweat lactate levels and blood lactate levels. This provides a tool for prognostic patient risk assessment. In particular, temporal changes in time lag may reflect changes in the patient's metabolism which are indicative of patient deterioration or improvement, particularly in critically ill patients with sepsis or liver dysfunction.

By way of illustration, Fig. 14 schematically shows two data series of lactate concentration in blood and sweat respectively. Both sweat lactate measurements and blood lactate measurements could be separately acquired at a series of time points across a common measurement period. The time series for each may then be plotted, and their trends over time temporally registered with respect to one another to identify a time lag between them, as illustrated in Fig. 14.

Further to this, identifying lactate lag time is useful in subsequently identifying a cause of changes in the (D- and/or L-) lactate concentration in sweat. For example, if a patient's L-lactate level is detected to start rising 15 minutes after the start of a physiotherapy session, while it is known that his L-lactate lag time is 15 minutes, then it is very likely that the physiotherapy caused this rise.

Once a transient lactate signal (D-lactate upward inflection or D-/L-lactate ratio upward inflection) is detected by the sweat sensor device, the time lag can be determined and better correlation to blood lactate level. According to studies, if the intravenous solution contains a racemic mixture, there can be expected to be a maximum 13-fold increase in plasma D-lactate concentration and 2-fold increase in plasma L-lactate concentration, such that especially the ratio of D-to-L-lactate should be a plausible metric to be used for reference point calibration. Return to pre-infusion D- and L-lactate concentration occurred within a few hours of stopping the treatment. Reference is made in particular to the paper: Kuze S, Naruse T, Yamazaki M et al. Effects of sodium L-lactate and sodium racemic lactate on intraoperative acid base status. Anesth Analg 1992; 75: 702-07. Reference is also made to the paper: Delman K, Malek S, Bundz S et al. Resuscitation with lactated Ringer's solution after hemorrhage: lack of cardiac toxicity. Shock 1996; 5: 298-303.

The above technique for measuring the lactate time lag can be easily integrated into a clinical workflow at the patient bedside or during surgical procedures, in particular since it is common to provide one or multiple lactate Ringer's infusions in such clinical circumstances.

The methodology could be further supplemented in some examples by a means for temporal synchronization between provision of a lactate injection or infusion and the lactate measurement series being acquired with the sweat measurement device. For example, by scanning a patient code attached to an infusion bag (when the infusion is given) or by pushing a button which triggers a wireless transmission, synchronization of D-/L-lactate injection/infusion with the sweat device could be triggered. Alternatively, in some examples automated detection of injections can be accomplished using just the lactate measurements, particularly in clinical scenarios where there is a negligible or very short uptake time of lactate, for example by detecting a sudden sharp rise in D-lactate which is preceded by a gradual decreasing trend in D-lactate.

Thus in summary, lactate exists in the body as racemic mixture of D- and L- lactate. In the above described embodiment, it is proposed to make use of these two chemical forms of lactate. Normally only L-lactate is measured clinically as concentrations are 100 times higher than D-lactate. However, by administering controlled racemic Ringer's solution (or D-lactate) it is possible to artificially increase the D-lactate to gain additional clinical information regarding uptake and diffusion time.

Within this technical field, it is generally considered that measurements every 1-2 hours are sufficient in most acute cases, and provides clinically relevant data about the decrease in lactate levels in critically ill patients (giving information about potential sepsis, or about cardiac resuscitation). However, if semi-continuous sweat lactate monitoring is done, dynamic or adaptive re-calibration of the sensor system can be realized if multiple injections or infusions are given and either the recurring transient peaks or inflection points are used to determine time lags, as outlined above.

The above described procedure for determining lactate uptake lag time may be further supplemented or varied in a variety of ways, as outlined below. These adaptations may enable the lag time measurements to be made more specific and reliable.

In some embodiments, a time derivative of the D/L lactate ratio or L/D lactate ratio, or the D-Lactate level, may be used instead of the raw signal.

In some examples, the method may further comprise monitoring a ratio of D-lactate to sodium [Na$^+$]. A change in the D lactate levels alone could be indicative merely of a change in sweat rate, i.e. increased sweat fluid volume leading to increase in concentration of D lactate. By monitoring the ratio of the lactate to sodium (where sodium is an indicator of sweat levels), this allows for identifying changes in D lactate which may be due to sweat level changes. This enhances robustness of the measurements of the lactate levels. For example, it has been found that the D-lactate concentration in sweat samples tripled after short periods of running but the relative concentration to sodium ion concentration was not altered.

It is further noted that although the above example method has been described in terms of measuring lactate, the same principle can be applied for measuring delay times in uptake of a stereoisomer of any other molecule. For example, almost all (naturally produced) amino-acids (e.g. alanine, lysine etc), have L-/D stereoisomer configurations.

Furthermore, the stereoisomer concentration (whether of lactate or a different molecule) does not have to be measured in sweat, it may be measured in any other biofluid.

Accordingly, more generally an aspect of the invention may comprise a system comprising:

a body fluid molecule stereoisomer concentration sensor (for instance as described in any of the embodiments discussed above); and
a controller adapted to:

obtain from the sensor a data series of stereoisomer concentration measurements for a subject, over a continuous measurement session;
detect, in the measurement series, timings of either positive or negative inflection points in the data series;
receive a data input indicative of timings of one or more stereoisomer injection events administered to the subject;
determine a lag time between at least one stereoisomer injection event and a temporally closest inflection point following the stereoisomer injection event.

There will now be described a further method employing use of body lactate measurements for obtaining further clinically relevant information.

It should be noted that the method described below is not necessarily limited to the use of any of the example sensor devices described previously in this disclosure. In general, the D and/or L-lactate measurements could be derived from any suitable means, for example D- and L-lactate sensitive electrochemical sensors, or capture surfaces. Capture surfaces provide an alternative sensing means using Ab-Ag binding and ELISA binding kinetics. However, the two main optical approaches described previously are preferred since they are simpler, faster, and allow for greater measurement frequency (e.g. semi-continuous). For example, alternative approaches require reagent replenishment and capture surface regeneration.

The below outlined method provides a means for establishing a conversion factor for converting measured sweat lactate concentration levels to an equivalent estimate for blood lactate levels.

Blood lactate levels are a well-known and well-established biomarker, and clinicians are used to working with and interpreting blood lactate concentrations. However, as discussed above blood lactate measurements take significant time to return results, since the samples need to be sent to a lab, and the method of obtaining the blood sample is invasive in nature. Measurement of sweat lactate on the other hand is non-invasive and fast. However, sweat lactate levels are less familiar as a biometric to clinicians. In order for the clinicians to more easily interpret these values, a translation to equivalent blood lactate concentration would be of value.

For this purpose, it is necessary inter alia to take into account the following three factors.

First, in common with any other biomarker, lactate in blood is not directly excreted through sweat. There is a certain conversion factor, c, which depends upon the kind of biomarker.

Second, in the particular case of L-lactate, there is an extra complexity due to that the fact that the sweat glands themselves produce L-lactate. When sweat rate is higher, sweat lactate levels increase. Current clinical theory suggests that when the sweat rate is higher, the sweat glands are required to work harder, and therefore produce more L-lactate.

Third, for purposes of deriving the conversion factor, it will be assumed that sweat lactate scales linearly with blood, i.e., lactate clearance through the kidneys follows the same kinetics as clearance via sweat glands.

With regards to the second consideration above, for simplicity, the L-lactate originating from the sweat glands themselves will not be included in below calculations as it can be determined via other well-known methods, for example as described in WO 2019/051107. It will be assumed in other words that this sweat-rate independent part has already been determined and the below derivations will not take into account L-lactate produced by the sweat glands. In some examples, a correction may be made for sweat-gland produced lactate, which may be performed in dependence upon the sweat rate measured). For instance, a high sweat rate is relevant for athletes, while for clinical patients at risk of sepsis after surgery we a low, natural sweat rate can be assumed since these patients are mainly in a sedentary state.

What is therefore sought is a conversion factor c between sweat and blood lactate measurements. If c is known, the concentration of L-lactate in blood can be calculated from the measured concentration in sweat.

The method proposed below employs use of one or more lactate injections, and the acquisition of sweat and blood lactate measurements before and after the injection(s).

In the calculations that follow, the following abbreviations will be used:

$c$: conversion factor of sweat L-lactate to blood L-lactate;

$BOL$: concentration of body-produced L-lactate in blood;

$BRL$: concentration of L-lactate in blood originating from the lactate solution (e.g. Ringer's solution);

$BL$: total concentration of L-lactate in blood (body-produced plus that originating from the lactate solution);

$SOL$: concentration of body-produced L-lactate in sweat;

$SRL$: concentration of L-lactate in sweat originating from the lactate solution

$S$: total concentration of L-lactate in sweat (body-produced plus that originating from the lactate solution)

*before* (as subscript): at a time just before the lactate solution is introduced to the body (or at least before it can be detected in sweat);

*after* (as subscript): just after the lactate solution has been introduced to the body (and has found its equilibrium in sweat).

Following the above definitions, the total sweat concentration of L-lactate can be defined as follows:

$$S = c \cdot BL \tag{1}$$

Just before the lactate solution is injected:

$$S_{before} = c \cdot BOL \tag{2}$$

Just after the lactate solution has been injected:

$$S_{after} = c \cdot (BOL + BRL) \tag{3}$$

Equation (3) minus equation (2) yields:

$$S_{after} - S_{before} = c \cdot BRL \tag{4}$$

and thus

$$c = (S_{after} - S_{before})/BRL \tag{5}$$

$S_{after}$ and $S_{before}$ are the measured sweat concentrations (and thus known) and BRL can be estimated as:

$$BRL = I \cdot V_i / V_b \tag{6}$$

where $I$ is the concentration of L-lactate in the lactate solution, $V_i$ is the volume of the Ringer's solution applied (within a certain time interval), and $V_b$ is the blood volume of the patient. For an adult, $V_b$ is on average 5 liters.

Better estimations can be made by including knowledge of gender, height, weight or body-mass-index, e.g. by using the Nadler equation or the Lemmens-Bernstein-Brodsky equation. Reference is made for example to the paper: Ragav Sharma and Sandeep Sharma, Physiology, Blood Volume, StatPearls . Treasure Island (FL): StatPearls Publishing; 2020 Jan, 2020 Apr 25.

Pregnancy, hypovolemic shock, and edema also influence the blood volume and thus might also be taken into account for a better estimation.

Using the determined conversion factor c, the sweat concentration values can be converted to (estimated) blood concentration values. Blood lactate concentration is a biomarker with which clinicians are already familiar. Using the sweat lactate measurements carries the advantage that time delays waiting for blood lactate test results to return from the lab can be avoided, and the invasive procedure of blood sample extraction does not need to be performed.

Thus, following the principles above, one or more embodiments propose to provide a method comprising the following steps:

obtaining a first sweat lactate measurement (which may be D-lactate, L-lactate or a ratio of the two);
obtaining a second sweat lactate measurement, where the second measurement follows administration of a lactate injection to the subject;
determining a conversion factor, c, between sweat L- or D-lactate and blood L- or D-lactate by computing the following equation:

$$c = (S_{after} - S_{before})/(I \cdot V_i / V_b) \qquad (7)$$

where c is the conversion factor from sweat L-lactate or D-lactate lactate concentration to blood L-lactate or D-lactate concentration, $S_{before}$ is the lactate concentration of the lactate of interest in sweat before the lactate injection, $S_{after}$ is the lactate concentration of the lactate of interest in sweat after the lactate injection, $I$ is the concentration of the lactate of interest, i.e., L-lactate or D-lactate in the lactate injection solution, $V_i$ is the total volume of the lactate solution injected, and $V_b$ is the total blood volume of the subject.

The sweat lactate measurements may be obtained using a sweat lactate sensor in accordance with any of the embodiments or examples outlined above, or in accordance with any claim of this application. Alternatively, any other source of sweat lactate measurements may be used.

According to one or more embodiments of at least one aspect of this invention, there may be provided a system comprising a sweat sensor in accordance with any of the embodiments or examples outlined above, and further comprising a controller or processor adapted to perform the steps of the method outlined immediately above, relating to deriving the conversion factor, c, between sweat lactate concentration and blood lactate concentration.

As noted above, the above calculation assumes a linear mathematical relationship between blood and sweat lactate. However, in other examples, the method may be adapted so that the relationship between sweat lactate and blood lactate is modelled according to a different mathematical relationship, for example a first order polynomial relationship ($y = mx + nx^2$). Thus, the estimation of the conversion factor c is not limited to an assumption of a linear mathematical relationship and, in general, any type of mathematical relationship might be used, based on either theoretical modelling or experimental observation. It is found that a linear relationship assumption produces good and reliable results. However, without wishing to be bound by theory, it is possible that a different type of relationship might yield superior results and therefore the invention is not limited to this assumption.

It is noted that although the embodiment described above is specifically for detecting a conversion between sweat and blood concentrations of L or D lactate, the same method can also be applied to stereoisomers of other molecules which are present in both sweat and blood. For example, almost all (naturally produced) amino-acids (e.g. alanine, lysine etc), have L-/D stereoisomer configurations.

By way of example, bilirubin is known to be detectable in both blood and sweat. By way of example, reference is made to the paper: Ebbesen, F., Madsen, P., Vandborg, P. et al. Bilirubin isomer distribution in jaundiced neonates during phototherapy with LED light centered at 497 nm (turquoise) vs. 459 nm (blue). Pediatr Res 80, 511-515 (2016). This paper describes one isomer of Bilirubin being more prominently present when light therapy is administered with a first wavelength, and the other isomer being more prominently present when therapy is administered with a second wavelength. Thus it can be seen that the relative concentrations of the Bilirubin stereoisomers vary according to circumstances.

By way of further example, also non-body-originating molecules could be detected, e.g. stereoisomers of molecules present in, or generated by, certain pharmaceuticals. Reference is made for example to the paper: Int J Appl Basic Med Res. 2013 Jan-Jun; 3(1): 16-18.

Thus more generally, an aspect of the invention may provide a system comprising:

a sweat sensor for sensing a concentration of at least one stereoisomer of a predefined molecule in a sweat sample (for example a sensor as described in embodiments earlier in this disclosure); and
a controller adapted to:

obtain from the sensor a first measurement of a concentration of a stereoisomer of interest,
obtain from the sensor a second measurement of a concentration of the stereoisomer of interest, where the second measurement follows administration of a stereoisomer injection to the subject of the stereoisomer of interest; and

determine a conversion factor, c, between a sweat concentration of the stereoisomer and a blood concentration of the stereoisomer by computing the following equation:

$$c = (S_{after} - S_{before})/(I \cdot V_i / V_b)$$

where c is the conversion factor from the sweat stereoisomer concentration to blood stereoisomer concentration, $S_{before}$ is the concentration of the stereoisomer of interest in sweat before the stereoisomer injection, $S_{after}$ is the stereoisomer concentration of the stereoisomer of interest in sweat after the stereisomer injection, I is the concentration of the stereoisomer of interest in the stereoisomer injection solution, $V_i$ is the volume of the stereoisomer solution injected, and $V_b$ is the blood volume of the subject.

For the specific example discussed above for use in measuring lactate, then the stereoisomer of interest may be D-Lactate or L-lactate. The injection may be an injection of a D-Lactate or L-lactate solution, e.g. Ringer's solution.

Embodiments outlined above employ use of a controller. The controller may comprise one or more processors.

Reference to processor in this disclosure may be reference to a single processor or a plurality of processors. A processor may be located in a single containing device, structure or unit, or it may be distributed between a plurality of different devices, structures or units. Reference therefore to the processor being adapted or configured to perform a particular step or task may correspond to that step or task being performed by any one or more of a plurality of processing components, either alone or in combination. The skilled person will understand how such a distributed processing arrangement can be implemented. A processing arrangement may be provided which includes a communication module or input/output for receiving data and outputting data to further components.

The one or more processors can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A body fluid sensor (12) comprising:

   a body fluid collection system (16);
   a polarized light source (20) for directing analysis light to a collected body fluid sample;
   at least one polarization state modifier (22);
   a light detector (24); and
   a processor (26) for processing the detected light,

wherein, during operation, the polarization state modifier and the collected body fluid sample are along a common optical path (32) between the light source and the light detector,

and wherein the processor is adapted to derive an optical property of the detected light which has been modified by a polarization modification induced by the body fluid sample, and thereby determine a concentration of at least a first stereoisomer of a predefined molecule in the body fluid sample, or a ratio between a concentration of a first stereoisomer of a molecule and a concentration of a second stereoisomer of the molecule present in the body fluid sample,

wherein the polarization state modifier is arranged optically upstream from the sample, between the polarized light source and the sample, and

wherein the optical property determined by the processor is a polarization modification induced by an optical activity of the body fluid sample.

2. A sensor (12) as claimed in claim 1, wherein the first stereoisomer is D-Lactate and the second stereoisomer is L-lactate.

3. A sensor (12) as claimed in claim 1 or 2, wherein the body fluid is sweat.

4. A sensor (12) as claimed in any of claims 1-3, wherein the polarization state modifier (22) is adapted to apply a set of different polarization rotation angles.

5. A sensor (12) as claimed in any of claims 1-4, wherein the sensor comprises a controller adapted to control the polarized light source (20) for generating analysis light of at least two different wavelengths, and wherein the processor (26) is adapted to derive individual concentrations of the first and second stereoisomer of the molecule from the measurement of the optical property at different of the two or more wavelengths.

6. A sensor (12) as claimed in any of claims 1-5, wherein the body fluid collection system (16) comprises a fluid conduit subsystem (18), and wherein the fluid sample is contained in the fluid conduit subsystem during analysis.

7. A sensor (12) as claimed in claim 6, wherein the at least one polarization state modifier (22) is comprised by one or more walls of at least one fluid conduit of the fluid conduit subsystem (18).

8. A sensor (12) as claimed in claim 7, wherein the polarization state modifier (22) is imprinted on the wall of said at least one fluid conduit.

9. A sensor (12) as claimed in any of claims 6-8, wherein the polarization state modifier (22) comprises a plurality of segments (23) with different polarization angles along the fluid conduit subsystem (18) and wherein the light detector (24) is configured to separately detect light that has passed through different segments.

10. A sensor (12) as claimed in claim 9, wherein the segments (23) are arranged such that, during operation, all simultaneously lie in at least one optical path extending from the light source (20) to the detector (24) via the body fluid sample in the fluid conduit subsystem (18).

11. A sensor as claimed in any of claims 6-10, wherein a fluid flow channel (19) defined by at least one conduit of the fluid conduit subsystem (18) is arcuate, the arc of the conduit lying in a plane, and wherein said common optical path has a directional component which is perpendicular to said plane.

12. A body fluid sensor (12) comprising:

a body fluid collection system (16);
a polarized light source (20) for directing analysis light to a collected body fluid sample;
at least one polarization state modifier (22);
a light detector (24); and
a processor (26) for processing the detected light,
wherein, during operation, the at least one polarization state modifier (22) and the collected body fluid sample are along a common optical path (32) between the light source and the light detector,
and wherein the processor (26) is adapted to derive an optical property of the detected light which has been modified by a polarization modification induced by the body fluid sample, and thereby determine a concentration of at least a first stereoisomer of a predefined molecule in the body fluid sample, or a ratio between concentrations of at least a first and second stereoisomer of a predefined molecule present in the body fluid sample, wherein the at least one polarization state modifier (22) includes a birefringent element (72) optically upstream from the sample and a linear polarizer (74) optically downstream from the sample, and wherein the optical property determined by the processor (26) is a change in a central wavelength, $\delta\lambda$, of an optical spectrum of the detected light compared to an optical spectrum of the light generated by the light source (20).

13. A sensor (12) as claimed in claim 12,

wherein the polarized light source (20) is linearly polarized in a first direction, wherein the birefringent element (72) is arranged to receive the light generated by the light source and to output two linearly polarized light beams having respectively perpendicular polarization directions; and wherein the linear polarizer (74) has a direction of polarization perpendicular to the first direction.

14. A sensor (12) as claimed in claim 12 or 13, wherein the birefringent element (72) is a half wave plate.

15. A sensor (12) as claimed in any of claims 12-14, wherein

the birefringent element (72) comprises a birefringent material; or the birefringent element (72) comprises an electrically addressable liquid crystal polarizer arrangement.

16. A sensor (12) as claimed in any of claims 12-15, wherein the body fluid collection system (16) comprises a fluid conduit subsystem (18), and wherein the body fluid sample is contained in the fluid conduit subsystem during analysis, and

optionally wherein the at least one polarization state modifier (22) is comprised by one or more walls of at least one fluid conduit of the fluid conduit subsystem, and optionally wherein the at least one polarization state modifier is imprinted on at least one wall of said at least one fluid conduit.

17. A sensor (12) as claimed in claim 16, wherein a fluid flow channel (19) defined by at least one fluid conduit of the fluid conduit subsystem (18) is arcuate, the arc of the conduit lying in a plane, and wherein said common optical path between the light source (20) and the detector (24) has a directional component which is perpendicular to said plane.

18. A sensor (12) as claimed in any of claims 1-17, wherein the sensor is integrated in a wearable unit.

19. A system comprising:

a sensor (12) as claimed in any of claims 1-18; and a controller adapted to:

obtain from the sensor a data series of concentration measurements of the at least one stereoisomer for a subject, over a continuous measurement session; detect, in the measurement series, timings of either positive or negative inflection points in the data series; receive a data input indicative of timings of one or more stereoisomer injection events administered to the subject; determine a lag time ($\Delta T1$, $\Delta T2$) between at least one stereoisomer injection event and a temporally closest inflection point following the stereoisomer injection event.

20. A system comprising:

a sensor (12) as claimed in any of claims 1-18, wherein the sensor is a sweat sensor; and a controller adapted to:

obtain from the sensor a first measurement of a concentration of a stereoisomer of interest, obtain from the sensor a second measurement of a concentration of the stereoisomer of interest, where the second measurement follows administration of a stereoisomer injection to the subject of the stereoisomer of interest; and determine a conversion factor, c, between a sweat concentration of the stereoisomer of interest and a blood concentration of the stereoisomer of interest by computing the following equation:

$$c = (S_{after} - S_{before})/(I \cdot V_i / V_b)$$

where c is the conversion factor from the sweat stereoisomer concentration to blood stereoisomer concentration, $S_{before}$ is the concentration of the stereoisomer of interest in sweat before the stereoisomer injection, $S_{after}$ is the concentration of the stereoisomer of interest in sweat after the stereoisomer injection, $I$ is the concentration of the stereoisomer of interest in the stereoisomer injection solution, $V_i$ is the volume of the stereoisomer solution injected, and $V_b$ is the blood volume of the subject.

COO⁻

HO ▮▮▮▬-C◀━━▶ H

CH₃

L (+) lactate

COO⁻

H ◀━━▶ C -▮▮▮ OH

CH₃

D (−) lactate

FIG. 1

Incident polarized light

$I_{final} = I_0 \cos^2 \theta$

$E_0$

θ

Transmitted polarized light

FIG. 2

FIG. 3

FIG. 4

EP 4 206 650 A1

## Polarimetry configuration

**FIG. 5**

**FIG. 6**

**FIG. 7**

27

FIG. 8

FIG. 9

FIG. 10

FIG. 11

FIG. 12

b) IV infusion (continuous)

FIG. 13

FIG. 14

Europäisches Patentamt

European Patent Office

Office européen des brevets

# EUROPEAN SEARCH REPORT

Application Number

EP 21 21 7899

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | QIAO ZHEN ET AL: "-lactate based on optical weak measurement", ANALYTICAL METHODS, vol. 11, no. 16, 2019, pages 2223-2230, XP055930602, GB ISSN: 1759-9660, DOI: 10.1039/C9AY00121B | 1-5, 12-15 | INV. G01N21/21 |
| Y | * abstract * | 6-8,10, 11,16-18 | |
| A | * page 2224, left-hand column, last paragraph - right-hand column, paragraph 4; figures 1,2 * * page 2226, left-hand column, paragraph 2 - page 2229, left-hand column, last paragraph; figures 3-9 * | 9,19,20 | |
| Y | WO 2019/210240 A1 (ECCRINE SYSTEMS INC [US]) 31 October 2019 (2019-10-31) * paragraph [0015] * * paragraph [0029] - paragraph [0032]; figures 2-4 * * paragraph [0034] - paragraph [0037]; figures 5-6 * | 6,11, 16-18 | |
| Y | US 2021/000390 A1 (ROGERS JOHN A [US]) 7 January 2021 (2021-01-07) * paragraph [0031] - paragraph [0032]; figures 1-2 * * paragraph [0058] - paragraph [0067]; figures 28-37 * * paragraph [0269] - paragraph [0270] * * paragraph [0282] * * paragraph [0024] - paragraph [0348] * | 6,11, 16-18 | TECHNICAL FIELDS SEARCHED (IPC)  A61B |
| Y | US 2020/113441 A1 (VARGHESE BABU [NL] ET AL) 16 April 2020 (2020-04-16) * paragraph [0122] - paragraph [0124]; figures 8-9 * | 7,8,10 | |

The present search report has been drawn up for all claims

| Place of search | Date of completion of the search | Examiner |
|---|---|---|
| Munich | 20 June 2022 | Consalvo, Daniela |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**Application Number**

**EP 21 21 7899**

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

**see sheet B**

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

**1-18 (completely); 19, 20 (partially)**

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

1. claims: 1-18(completely); 19, 20(partially)

   Sensor for body fluid analysis based on polarimetry or optical quantum weak measurements.
   ---

2. claim: 19(partially)

   System adapted to perform a specific diagnostic method using measurements obtained during administration of lactate to a subject using the sensor of claims 1-8 or claims 10-18
   ---

3. claim: 20(partially)

   System adapted to perform a specific diagnostic method using measurements obtained during administration of lactate to a subject using the sensor of claims 1-8 or claims 10-18
   ---

## ANNEX TO THE EUROPEAN SEARCH REPORT
## ON EUROPEAN PATENT APPLICATION NO.

EP 21 21 7899

This annex lists the patent family members relating to the patent documents cited in the above-mentioned European search report.
The members are as contained in the European Patent Office EDP file on
The European Patent Office is in no way liable for these particulars which are merely given for the purpose of information.

20-06-2022

| Patent document cited in search report | Publication date | Patent family member(s) | Publication date |
|---|---|---|---|
| WO 2019210240 A1 | 31-10-2019 | NONE | |
| US 2021000390 A1 | 07-01-2021 | US 2017224257 A1<br>US 2021000390 A1<br>WO 2016025430 A1 | 10-08-2017<br>07-01-2021<br>18-02-2016 |
| US 2020113441 A1 | 16-04-2020 | BR 112019020719 A2<br>CN 110740679 A<br>EP 3384829 A1<br>EP 3606409 A1<br>JP 2020516879 A<br>RU 2019135280 A<br>US 2020113441 A1<br>WO 2018185212 A1 | 12-05-2020<br>31-01-2020<br>10-10-2018<br>12-02-2020<br>11-06-2020<br>05-05-2021<br>16-04-2020<br>11-10-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019051107 A

### Non-patent literature cited in the description

- **QIAO et al.** The real-time determination of D- and L-lactate based on optical weak measurement. *Anal. Methods,* 2019, vol. 11, 2223-2230
- **KUZE S ; NARUSE T ; YAMAZAKI M et al.** Effects of sodium L-lactate and sodium racemic lactate on intraoperative acid base status. *Anesth Analg,* 1992, vol. 75, 702-07
- **DELMAN K ; MALEK S ; BUNDZ S et al.** Resuscitation with lactated Ringer's solution after hemorrhage: lack of cardiac toxicity. *Shock,* 1996, vol. 5, 298-303
- **RAGAV SHARMA ; SANDEEP SHARMA.** Physiology, Blood. StatPearls Publishing, January 2020
- **EBBESEN, F. ; MADSEN, P. ; VANDBORG, P. et al.** Bilirubin isomer distribution in jaundiced neonates during phototherapy with LED light centered at 497 nm (turquoise) vs. 459 nm (blue). *Pediatr Res,* 2016, vol. 80, 511-515
- *Int J Appl Basic Med Res.,* January 2013, vol. 3 (1), 16-18